# FASCICULE DE BREVET EUROPEEN

(11) **EP 2 964 313 B1**
(45) Date de publication et mention de la délivrance du brevet: **07.10.2020**
(21) Numéro de dépôt: 14716925.4
(22) Date de dépôt: 04.03.2014
(51) Int. Cl.: A61M 39/10, A61M 39/20, A61M 39/16, A61M 39/18

(54) **ENSEMBLE CONNECTEUR CAPUCHON**
STECKER-SCHUTZDECKEL-EINHEIT
CAP CONNECTOR ASSEMBLY

(30) Priorité: 04.03.2013 EP 13157635
(43) Date de publication de la demande: 13.01.2016
(73) Titulaire: Debiotech S.A., 1004 Lausanne (CH)
(72) Inventeur: NEFTEL, Frédéric, CH-1005 Lausanne (CH)
(74) Mandataire: Weihs, Bruno Konrad
(86) Numéro de dépôt international: PCT/IB2014/059429
(87) Numéro de publication internationale: WO 2014/136050

(56) Documents cités:
- WO-A1-03/041789
- FR-A1- 2 468 059
- US-A- 3 976 311
- US-A1- 2012 042 971

## Description

### Domaine de l'invention

L'invention concerne un ensemble connecteur capuchon pouvant être utilisé dans de nombreux domaine d'activité qui nécessite un certain niveau d'aseptiedes connecteurs dont le domaine médical ainsi qu'une méthode de décapuchonnage. Ladite invention permet d'éviter certaine manipulation qui risquerait de contaminer ou souiller tout ou partie de la ligne fluidique comprenant ledit connecteur.

### Etat de la technique

Dans de nombreux domaine d'activité, il est parfois utilisé des connecteurs stériles ou propres (en particulier dans le domaine médical ou dans l'industrie par exemple en salle blanche) afin de relier deux lignes fluidiques qui doivent restées aseptiques ou propre (sans poussière par exemple). Ces connecteurs peuvent être munis de capuchons permettant de fermer les parties à protéger du connecteur afin d'éviter toute contamination ou que des saletés ne soient en contact avec certaines parties des connecteurs avant la connexion desdits connecteurs.

De façon générale lors de la connexion des deux connecteurs, la stérilité ou « propreté » doit être assurée sur au moins les parties protégées par le capuchon et l'utilisateur doit être vigilent quant à la stérilité ou « propreté » jusqu'à la connexion des connecteurs. Dit autrement, lorsque que les connecteurs sont décapuchonnés mais pas encore connectés, le praticien ou le patient doit manipuler avec une grande précaution afin de garantir qu'il n'a pas contaminé, de façon non intentionnelle, les connecteurs.

En outre, la connexion de deux lignes fluidiques peut nécessiter l'utilisation de deux connecteurs distincts, chacun étant monté sur une ligne fluidique. Ainsi pour les relier, il faut dans un premier temps décapuchonner les connecteurs puis, dans un deuxième temps, connecter lesdits connecteurs ensembles. Toutefois, afin de ne pas contaminer ou salir les connecteurs, une fois décapuchonnés les connecteurs ne doivent être reposés pendant le décapuchonnage de l'autre. Les deux connecteurs doivent ainsi être manipulés ensembles et il est indispensable de toujours maintenir un connecteur pendant le décapuchonnage de l'autre connecteur. Autrement dit, les actions de décapuchonnage doit s'effectuer alors que les deux connecteurs sont maintenus par l'utilisateur, rendant ainsi la tâche plus compliquée.

A titre d'exemple dans le domaine médical, des lignes fluidiques doivent être reliées entre elles par tout patient atteint d'insuffisance rénale et nécessitant une dialyse péritonéale à domicile. Afin d'éviter les risques de contamination, responsables de péritonites infectieuses, certains appareils (SleepSafe de Fresenius) préconisent la connexion automatisée. Toutefois le tiroir, où l'on installe les connecteurs afin d'assurer leur connexion automatisée, peut être non stérile, réalisant alors un risque accru de contamination. D'autres dispositifs sont équipés, sur la face externe du connecteur, d'une bague (système Baxter) permettant d'utiliser l'un des doigts de l'autre main (généralement l'auriculaire) pour retirer le capuchon tandis que chacune des mains tient respectivement une ligne à connecter. Un tel système ne garantit pas l'absence de contamination, étant donné la proximité des doigts avec la partie intérieur du connecteur et rends les manipulations difficiles à expliquer au patient.

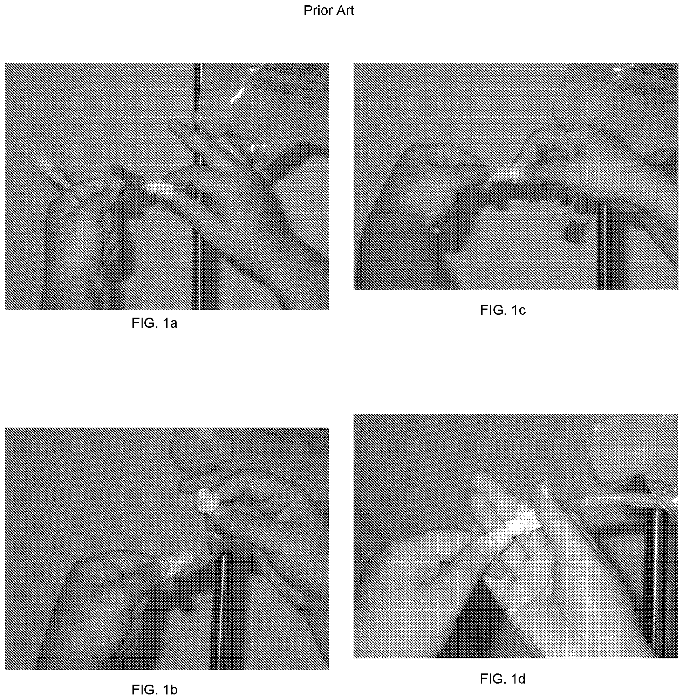

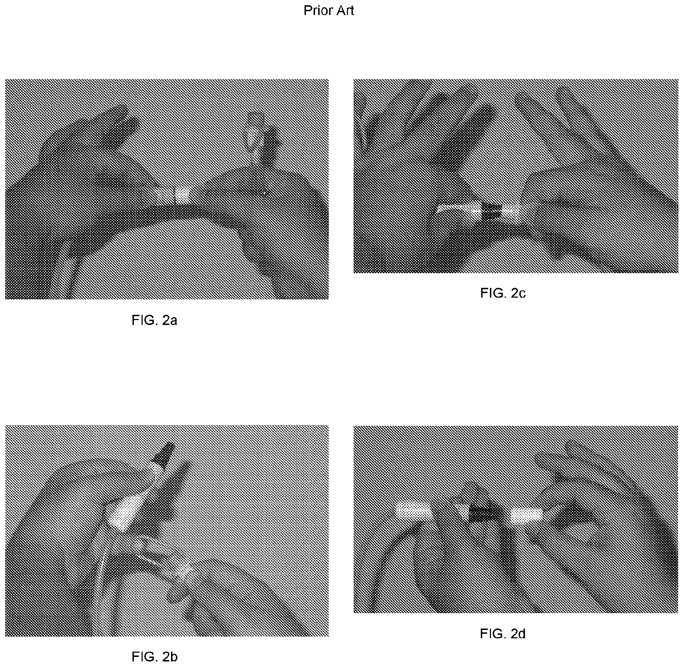

La demande PCT publiée sous le numéros WO 94/28855 A1 et le brevet US 4,573,980 A divulguent un capuchon permettant d'éviter le contact de tout élément avec la partie initialement protégée du connecteur. Ainsi, le capuchon comprend un anneau dans lequel un doigt peut y être glissé. Grâce à cet anneau, le connecteur peut être décapuchonné en tirant sur cet anneau. Ainsi, le praticien ne touche pas la partie protégée du connecteur. De façon plus détaillé, les figures 1a à 1c et 2a à 2c décrivent la manipulation nécessaire pour décapuchonner et connecter les connecteurs.

La figure 1a expose deux connecteurs, le premier est maintenu dans la main gauche du praticien et le deuxième dans sa main droite. Le deuxième connecteur est décapuchonné en maintenant ledit connecteur avec le pouce et l'index tandis que les autres doigts de la main droite restent éloignés dudit connecteur, ces doigts forment alors « une aile de papillon ». La main gauche maintien le premier connecteur tout en retirant le capuchon du deuxième connecteur. Dans la figure 1b, le premier connecteur est décapuchonné (grâce à l'anneau) par la main droite qui maintient en même temps le deuxième connecteur déjà décapuchonné. Dans la figure 1c, les deux connecteurs sont connectés en gardant les mains et doigts le plus éloigné possible de l'extrémité distale des connecteurs. La figure 1d expose une façon de connecter à proscrire car les doigts risquent de contaminer les connecteurs.

Les figures 2a à 2d exposent un autre ensemble connecteur/capuchon. Dans la figure 2a, lors du décapuchonnage du premier connecteur, la main gauche maintient le premier connecteur en formant une aile de papillon pendant que la main droite dévisse le capuchon du premier connecteur tout en maintenant éloigné le deuxième connecteur muni de son capuchon. Dans la figure 2b, le premier connecteur est décapuchonné et maintenu de façon à ne pas contaminer la partie initialement protégé par le capuchon tout en arrachant le capuchon du deuxième connecteur grâce à l'anneau de ce dernier avec un doigt de la main gauche, de préférence l'auriculaire. Dans la figure 2c, les deux connecteurs sont connectés en gardant les doigts des deux mains formant des ailes de papillon afin d'assurer de ne pas contaminer les connecteurs. Il est noté en figure 2d une manipulation à proscrire où les doigts de la main gauche peuvent contaminer la partie initialement protégée du premier connecteur lorsque celui-ci est décapuchonné.

En résumé, la demande PCT publié sous le numéros WO 94/28855 A1 et le brevet US 4,573,980 A divulguent un type de connecteur qui doit être maintenu par une main et décapuchonné par une autre, tout en faisant attention au placement de ces doigts. Ainsi, durant la manipulation, les risques de contaminations sont nombreux et la méthode de connexion contraignante voir laborieuse. Les praticiens doivent impérativement être formés pour ces manipulations qui ne sont pas évidentes.

Certains brevets revendiquent des ensembles connecteur/capuchon qui permettent de maintenir les connecteurs tout en les décapuchonnant. Par exemple, la demande PCT publié sous le numéro WO 83/00622 A1 ainsi que le brevet européen EP 0 621 053 B1 divulguent des capuchons fixés aux connecteurs par l'intermédiaire d'un moyen formant une charnière. Ces connecteurs peuvent être décapuchonnés simplement avec le pouce et être refermé. Cependant, le connecteur ne comprenant pas toujours de moyens de protection contre le contact du pouce avec la partie initialement protégée par le capuchon, lors de la manipulation, le pouce peut contaminer le connecteur. En effet, en poussant le capuchon, le pouce peut entrer en contact avec la partie initialement protégée ou proche de ladite partie et donc la contaminer. De plus, un tel système ne permet pas de savoir si le connecteur a déjà été utilisé ou simplement décapuchonné temporairement. Ainsi, le connecteur peut avoir été contaminé sans que le praticien ne le sache. Ce type de dispositif ne peut donc assurer que les connecteurs ne sont pas contaminés. En résumé, ce connecteur peut donc être maintenu et décapuchonné avec une seule main, toutefois les risques de contamination par le praticien ou le patient sont importants.

Un autre type de connecteur, tel que divulgué par la demande internationale Wo 03/041789 A1 ou US 2012/042971 A1, permet à l'utilisateur de ne pas toucher le capuchon car il reste maintenu au connecteur ou dans le connecteur lorsque les deux connecteurs sont connectés entre eux. Toutefois, étant donné que le capuchon reste connecté au connecteur ou dans l'enceinte du connecteur, il reste proche du chemin fluidique ce qui engendre un risque important de contamination direct ou indirect.

Une contamination indirecte doit être comprise comme une contamination de la ligne fluidique suite au fait qu'un élément contaminé soit proche de la ligne fluidique et puisse lui transmettre cette contamination. En particulier concernant les deux dispositifs des documents cités ci-dessus, les capuchons restent dans une enceinte proche de la ligne fluidique et ainsi peuvent transmettre une contamination à la ligne fluidique. Plus particulièrement, si les capuchons du dispositif de la demande Wo 03/041789 A1 ne se positionnent pas correctement dans l'espace prévu à cet effet, la ligne fluidique risque de heurter contre ces capuchons lors de la connexion des lignes fluidiques causant une détérioration dudit passage, un affaiblissement de l'étanchéité de ce passage et/ou une contamination du passage.

Fort de ce constat, de nouveau dispositif ont été créé en partant du postulat que la manipulation par le praticien est un facteur de risque important. Ainsi la demande PCT publiée sous le numéro WO 2009/006507 A1 divulgue un dispositif automatique qui permet de décapuchonner les connecteurs et de connecter les lignes fluidiques sans l'aide du praticien. Toutefois, il s'est avéré que ce genre de dispositif n'assurait pas la stérilité des connecteurs. En effet, les éléments qui protègent le système de connexion ne peuvent pas être aseptisé après chaque utilisation et donc ces éléments de protection peuvent engendrer une propagation de la contamination bien plus importante que le système manuel.

La manipulation par le praticien ou le patient reste ainsi le meilleur moyen de garantir la non-contamination de l'ensemble. Toutefois, la manipulation des dispositifs existants restent laborieuses et non évidentes engendrant de nombreux risque de contamination. De plus, certain dispositifs sont extrêmement complexe rendant leur réalisation trop couteuse sans pour autant garantir une propreté satisfaisante. Ainsi ces types de dispositif ne peuvent avoir de réalité économique.

La demande de brevet américaine US2012/004291 décrit un connecteur pour transférer un fluide comprenant un premier connecteur et un second connecteur. Chaque connecteur comprend une première partie externe comprenant une première partie obstructive et définissant un premier passage de fluide pour y recevoir un fluide. Chaque connecteur comprend en outre une première buse centrale couplée à la première partie externe. La buse centrale comprend un premier élément d'engagement. La première buse centrale est mobile entre une première position et une seconde position avec une rotation de la première partie externe. Dans la première position, la première partie obstructive et la première partie d'engagement se chevauchent sensiblement pour empêcher l'écoulement de fluide à travers le premier passage de fluide. Dans la seconde position, au moins l'un du premier élément d'engagement et du premier élément obstructif est déplacé pour permettre l'écoulement du fluide à travers le premier passage de fluide.

La demande de brevet internationale WO 03/041789 décrit un connecteur pour former une connexion stérile entre deux tubes, comprenant des première et seconde partie qui comprennent des conduits pouvant être raccordés et qui peuvent s'accoupler, des premier et second couvercle qui peuvent être retirés des parties assemblées pour exposer les extrémités connectables des premier et second conduit, de sorte que les conduits peuvent alors être connectés.

La demande de brevet française FR 2 468 059 décrit quant à elle un dispositif de connexion ou chaque embout est entouré par une jupe de forme sensiblement cylindrique raccordée à une collerette étant située en retrait par rapport à l'extrémité de connexion de l'embout qui est elle-même en retrait par rapport au bord de la jupe, le diamètre de la jupe entourant un embout étant différent du diamètre de la jupe entourant l'autre embout, chaque embout étant associé à un bouchon comportant un fond à partir duquel s'étend une jupe de forme générale cylindrique apte à être engagée avec étanchéité sur la jupe entourant ledit embout.

### Description générale de l'invention

La présente invention a pour objet de réaliser un connecteur stérile capuchonné dont la manipulation est simplifiée et le coût de fabrication limité, et permet d'éliminer les inconvénients des connecteurs connus cités plus haut.

La présente demande revendique la priorité de EP 13157635.7, déposée le 4 mars 2013 au nom de DEBIOTECH SA, dont le contenu intégral doit être considéré comme faisant partie de la présente demande.

La présente invention est décrite et caractérisée par la ou les revendications indépendantes, tandis que les revendications dépendantes décrivent d'autres caractéristiques de l'invention.

Dans le présent document, il faut comprendre le mot « enceinte » comme un espace dont les limites peuvent être matérialisées ou non et évolutive ou non. Une enceinte de sécurité représente ainsi un espace à l'intérieur duquel la propreté et/ou la stérilité doit être maintenu. L'enceinte de sécurité comprend le passage d'écoulement de fluide mais également un espace supplémentaire permettant d'éviter toute contamination indirecte causé par le capuchon ou la main de l'utilisateur durant la manipulation du connecteur. Dans un mode de réalisation, quand le capuchon est sur le connecteur, au moins une partie de la surface interne du capuchon délimite l'enceinte de sécurité et lorsque le connecteur est connecté à une ligne fluidique, l'enceinte de sécurité est délimitée par le connecteur et le dispositif auquel le connecteur est connecté. La surface externe du capuchon est en contact direct avec l'environnement extérieur, ainsi elle doit impérativement rester en dehors de l'enceinte de sécurité lorsque le capuchon ne protège pas le passage d'écoulement.

Ainsi, les capuchons permettent à la fois de protéger
les passages d'écoulement et d'empêcher la connexion des connecteurs. Ce mode de réalisation permet d'éviter la contamination directe et indirecte du passage d'écoulement car il permet de s'assurer que tout élément pouvant être contaminé (par exemple les capuchons, mains de l'utilisateur) soit suffisamment éloigné de l'enceinte de sécurité (et donc des passages d'écoulement de fluide) lors de la connexion des connecteurs entre eux. De plus, grâce à ce mode de réalisation, n'importe quel utilisateur comprend que lorsque les deux connecteurs sont connectés ensembles, la connexion fluidique est assuré et sécurisé.

### Liste des figures

Pour permettre une meilleure compréhension de l'invention, il sera décrit un ou plusieurs modes de réalisation illustrées par les figures jointes à ce document. Il va de soi que l'invention ne se limite pas à ces modes de réalisation.
**Figures 1a à 1d** **et** **2a à 2d** exposent l'utilisation de connecteurs de l'état de l'art.
**Figure 3** schématise un ensemble connecteur capuchon
**Figure 4a** schématise un système de connecteur
**Figures 4b à 4d** schématisent les actions nécessaire pour le décapuchonnage
**Figure 5** expose différents codages des moyens de couplage
**Figure 6** schématise un autre mode de réalisation d'un ensemble connecteur capuchon
**Figure 7** illustre un autre mode de réalisation d'un système de connecteur
**Figures 8a à 8c** exposent une différente vue du connecteur femelle et de son capuchon
**Figures 9a à 9c** exposent une différente vue du connecteur male et de son capuchon
**Figure 10** exposent un système de connecteur couplé par les capuchons
**Figure 11** schématise les actions nécessaire pour le décapuchonnage et la connexion des connecteurs
**Figure 12** exposent un mode de réalisation d'un système de connexion en vue éclatée
**Figure 13** montre un coupe des éléments exposés en figure 12
**Figures 14** **a et b** exposent une coupe d'un système de connexion avec capuchon couplé et une coupe d'un système de connexion connecté après avoir ôté les capuchons

### Liste des références numériques utilisées dans les figures

- 1: Ensemble connecteur capuchon
- 2: Ligne fluidique
- 3: Connecteur
- 4: Capuchon
- 5: Extrémité distale du connecteur
- 6: Extrémité proximale du connecteur
- 7: Extrémité distale du capuchon
- 8: Extrémité proximale du capuchon
- 9: Corps du connecteur
- 10: Corps du capuchon
- 11: Moyens de couplage.
- 12: Système de connecteur
- 13: Moyens de verrouillage
- 14: Moyens de retenu
- 15: Moyens de libération
- 16: Déclencheur
- 101: Ensemble capuchon / connecteur femelle (premier ensemble)
- 102: Ensemble capuchon / connecteur male (deuxième ensemble)
- 103: Connecteur femelle (premier connecteur)
- 104: Connecteur male (deuxième connecteur)
- 105: Capuchon du connecteur femelle (premier capuchon)
- 106: Capuchon du connecteur male (deuxième capuchon)
- 107: Corps du connecteur
- 108: Membrane
- 109: Filetage
- 110: Passage
- 111: Extrémité proximale
- 112: Extrémité distale
- 113: Ailette
- 114: Ergot
- 115: Elément cylindrique
- 116: Moyens de couplage
- 117: Fente
- 118: Lame élastique
- 119: Butée
- 120: Elément faisant saillie sur la lame élastique
- 121: Extrémité distale du capuchon
- 122: Extrémité proximale du capuchon
- 123: Axe principale
- 124: Butée
- 201: Ensemble capuchon / connecteur femelle (premier ensemble)
- 202: Ensemble capuchon / connecteur male (deuxième ensemble)
- 203: Connecteur femelle (premier connecteur)
- 204: Connecteur male (deuxième connecteur)
- 205: Capuchon du connecteur femelle (premier capuchon)
- 206: Capuchon du connecteur male (deuxième capuchon)
- 207: Corps du connecteur
- 208: Membrane
- 209: Moyens de connexion physique
- 210: Moyens de connexion fluidique
- 211: Extrémité proximale du connecteur
- 212: Extrémité distale du connecteur
- 213: Moyens de verrouillage du capuchon
- 214: Moyens de verrouillage du connecteur
- 215: Extrémité distale du capuchon
- 216: Extrémité proximale du capuchon
- 217: Dispositif de retenu du capuchon
- 218: Moyens d'appréhension
- 219: Passage d'écoulement de fluide
- 220: Tube

### Description détaillée de l'invention

Dans le présent document, la description détaillée de l'invention comporte des modes de réalisation de dispositifs, de systèmes et de méthodes présentés à titre d'illustration. Il est bien entendu que d'autres modes de réalisation sont envisageables et peuvent être apportées sans s'écarter de la portée ou l'esprit de l'invention. La description détaillée qui suit, par conséquent, ne doit pas être prise dans un sens limitatif.

Sauf indication contraires, les termes scientifiques et techniques utilisé dans le présent document ont des significations couramment utilisés par l'homme du métier. Les définitions apportées dans ce document sont mentionnées afin de faciliter la compréhension des termes fréquemment utilisés et ne sont pas destinées à limiter la portée de l'invention.

Les indications de direction utilisées dans la description et les revendications, telles que "haut", "bas", "gauche", "droite", "supérieur", "inférieur", et autres directions ou orientations sont mentionnées afin d'apporter plus de clarté en référence des figures. Ces indications ne sont pas destinées à limiter la portée de l'invention.

Dans le présent document, le terme « extrémité distale » doit être compris comme l'extrémité la plus éloigné d'une référence. En particulier, si la référence est la ligne fluidique du connecteur, l'extrémité distale d'un connecteur est l'extrémité qui est la plus éloigné de sa ligne fluidique. Cette extrémité peut être également qualifiée d'extrémité « libre » car contrairement à l'extrémité proximale, elle n'est pas directement reliée à la ligne fluidique. En outre, l'extrémité distale d'un capuchon est l'extrémité qui est la plus éloigné de la ligne fluidique.

Inversement, le terme « extrémité proximale » doit être compris comme l'extrémité la plus proche d'une référence. Ainsi, l'extrémité proximale d'un connecteur est l'extrémité qui est directement reliée à sa ligne fluidique et l'extrémité proximale d'un capuchon est l'extrémité qui est le plus proche de la ligne fluidique du connecteur.

Ainsi, lorsque le capuchon recouvre au moins l'extrémité distale du connecteur, l'extrémité distale du capuchon et l'extrémité proximale du connecteur représente les extrémités opposées de l'ensemble connecteur/capuchon. Préférentiellement, les extrémités distales des connecteurs sont façonnées de manière à rendre possible la connexion entre deux connecteurs compatible. Dans un mode de réalisation, cette connexion n'est possible que lorsque les capuchons sont retirés de l'extrémité distale des connecteurs.

Le terme praticien doit être compris au sens large comme celui qui est formé à manipuler les connecteurs et comprends aussi bien un membre du corps médical qu'un patient éventuel.

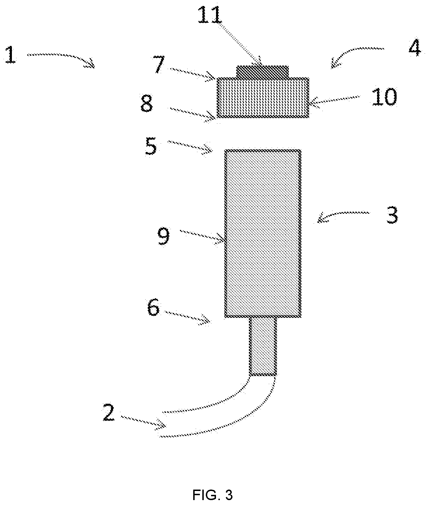

Dans un mode de réalisation exposé en figure 3, l'ensemble connecteur/capuchon (1) comprend un connecteur (3) et un capuchon (4). Ledit connecteur (3) comporte un corps (9) définissant un passage d'écoulement de fluide protégé par ledit capuchon (4) disposé à l'extrémité distale (5) dudit connecteur (3). L'ensemble (1) comprend des moyens de verrouillage (non exposés sur la figure 1) conçus pour maintenir au moins temporairement et solidement le capuchon (4) au connecteur (3). Le capuchon (4) comprend en outre des moyens de couplage agencés de manière à coupler ledit capuchon avec un élément comportant également des moyens de couplage. Préférentiellement, lesdits moyens de couplage (11) permettent un couplage au moins pendant que le praticien désactive les moyens de verrouillage afin de décapuchonner ledit connecteur (3) sans avoir à « toucher » le capuchon (4).

Dans un mode de réalisation, le capuchon (4) se couple à un autre dispositif ou système. Par exemple, si le connecteur est utiliser dans le cadre d'un traitement de dialyse, le dispositif de dialyse peut comprendre également des moyens de couplage de sorte que le capuchon (4) se souple sur le dispositif de dialyse afin de permettre le décapuchonnage dudit connecteur.

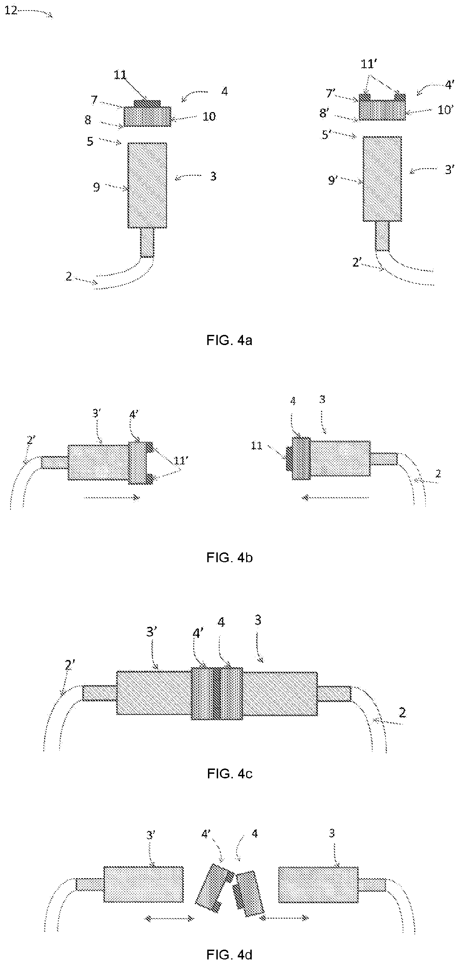

Dans un mode de réalisation exposé en figure 4a, un système (12) de connexion comprend deux connecteurs distincts (3, 3') permettant de relier deux lignes fluidiques (2, 2'). Les extrémités distales (5, 5') desdits connecteurs (3, 3') sont conçues pour permettre la connexion desdits connecteurs (3, 3'). En outre, lesdites extrémités distales (5,5') peuvent être de formes identiques ou de formes différentes mais compatible, par exemple, l'un de type male et l'autre de type femelle. Les connecteurs comprennent des moyens de connexion (non montrés sur les figures 4) de manière à permettre le maintien des deux connecteurs. Les capuchons (4, 4') comprennent des moyens de couplage (11, 11') façonnés de manière à coupler les deux capuchons (4, 4') ensembles. Les figures 4b à 4d décrivent la manipulation pour le décapuchonnage des connecteurs. En figure 4b, le praticien peut maintenir un connecteur dans chaque main par le corps (9, 9') desdits connecteurs (3, 3'). Les deux connecteurs (3, 3') doivent se rapprocher afin que (en fig 4c) les deux capuchons (4, 4') se couplent ensemble. Les moyens de connexion physique peuvent être opérants ou utilisés qu'après décapuchonnage des connecteurs.

Les corps (9, 9') peuvent comprendre des moyens d'appréhension permettant au praticien de tenir les connecteurs par les corps.

Dans un mode de réalisation, les connecteurs sont formés d'une unique pièce comprenant un corps :
- conçu pour être appréhendé avec la main d'un praticien, et
- définissant un passage d'écoulement de fluide.
Le corps peut également comprendre des moyens de connexion, des éléments de verrouillage (pour maintenir les connecteurs connectés) et/ou des moyens de verrouillage (pour maintenir les capuchons contre les connecteurs). Cette unique pièce peut être obtenue par un procédé de fabrication tel que l'injection, le moulage, le frittage ou l'usinage.

Dans un mode de réalisation, un ensemble (1) peut comprendre en outre des moyens de libération (non exposé) qui s'activent dès que les capuchons entrent en contact et/ou se couplent de manière à libérer au moins un capuchon de son connecteur (3, 3').

Dans un mode de réalisation, le mécanisme de déverrouillage est activé par le praticien, par exemple grâce à un mouvement de rotation d'au moins un connecteur (3, 3') sur son axe principale.

Dans un mode de réalisation, après avoir déverrouillé les moyens de verrouillage d'au moins un ensemble, le praticien laisse tomber au moins un capuchon. La figure 4d schématise des capuchons (4, 4') qui ne restent pas couplés après le déverrouillage. Toutefois, dans un mode de réalisation, les moyens de couplage (11, 11') peuvent permettre de maintenir les capuchons solidaires au moins temporairement après le déverrouillage des capuchons (4, 4').

Lesdits moyens de couplages (11, 11') peuvent être des aimants et/ou être façonnés comme un crochet ou une autre forme permettant le couplage desdits capuchons entre eux.

Dans un mode de réalisation, les moyens de couplage (du ou des capuchons ou dudit dispositif cité en exemple plus haut) comportent un système de codage de manière à ce que seuls les codages compatibles permettent au capuchon de se coupler. Eventuellement le dit codage permet à certains capuchons de se coupler à une série d'autres capuchons, tandis que certains capuchons ne peuvent se coupler qu'à un nombre plus limités de capuchons.

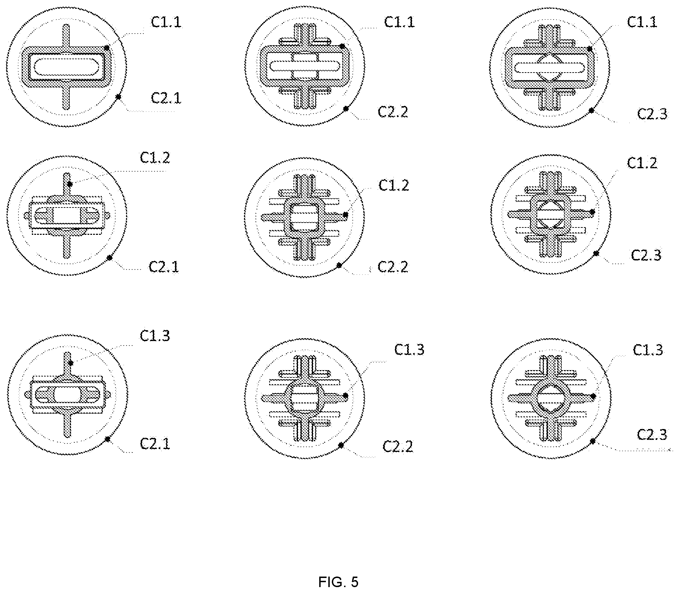

La figure 5 expose les différentes combinaisons possibles entre les différents moyens de couplage. Ainsi, les moyens de couplage C1.1 sont compatible avec les moyens de couplage du capuchon et/ou autre système/dispositif C2.1, C2.2 et C2.3. Cela signifie que le connecteur disposant d'un capuchon ayant des moyens de couplage de type C1.1 peut se connecter avec un système et/ou connecteur disposant d'un capuchon ayant des moyens de couplage de type C2.1, C2.2 ou C2.3. De même, C1.2 est compatible avec C2.2 et C2.3 mais incompatible avec C2.1. Et C1.3 est compatible avec C2.3 mais incompatible avec C2.1 et C2.2.

Selon un mode de réalisation, les moyens de couplage sont agencé de manière à s'emboité, par exemple un premier moyen de couplage comprend un élément femelle et un deuxième moyen de couplage comprend un élément mal compatible avec le premier moyen de couplage. Préférentiellement, les moyens de couplage sont conçus pour résister à des contraintes de rotation.

Ce codage permet ainsi d'éviter toute erreur de connexion car les codages incompatibles ne permettent de décapuchonner les connecteurs et donc, dans un mode de réalisation, les codages incompatibles rendent impossible la connexion des connecteurs. Par exemple, dans le cas où un connecteur comprend des moyens de connexion qui est de type femelle et l'autre (connecteur) de type male, un capuchon de connecteur comprenant des moyens de connexion de type male ne pourrait se coupler avec un autre capuchon de connecteur comprenant des moyens de connexion de type male. Autre exemple, lorsque deux lignes fluidiques ne peuvent être compatible alors le système de codage peut empêcher la connexion de ces deux lignes fluidiques ensemble car il serait impossible d'enlever les capuchons.

En plus du système de codage qui permet le couplage, l'ensemble connecteur capuchon peut comprendre des éléments de sécurité. Ces éléments de sécurité devant être levés (neutralisés) pour déverrouiller les capuchons. Aussi, le système de codage permet de neutraliser l'élément de sécurité de sorte que même si en forçant le praticien arrive à les coupler, le système de codage empêche de lever la sécurité.

Dans un mode de réalisation, au moins un connecteur (3, 3') et/ou au moins un capuchon (4, 4') comprend des moyens d'entrainement de manière à éloigner au moins partiellement le capuchon du connecteur lorsque les moyens de verrouillage sont désactivés. Ces moyens d'entrainement peuvent permettre d'éjecter le capuchon de sorte que le praticien n'ait besoin de n'effectuer aucune autre manipulation pour libérer le connecteur ou simplement de déplacer le capuchon afin que le praticien n'ait qu'à orienter l'extrémité distale du connecteur vers le bas pour que le capuchon tombe.

Dans un mode de réalisation, les moyens de verrouillage sont détruits grâce à une manipulation du praticien de sorte que le capuchon se désolidarise du connecteur. Ce mode de réalisation permet de ne pas re-capuchonner des connecteurs déjà utilisés, de sorte qu'il soit facile et évident de savoir quand un connecteur a déjà été utilisé ou simplement que le capuchon a déjà été enlevé (ce qui ne garantit pas la propreté du connecteur).

Dans un mode de réalisation, les moyens de verrouillage comprennent au moins un élément de sécurité de manière à empêcher la désactivation non intentionnelle des moyens de verrouillage. Cet élément de sécurité peut nécessiter une force minimum ou une manipulation spécifique pour être désactivé / retiré.

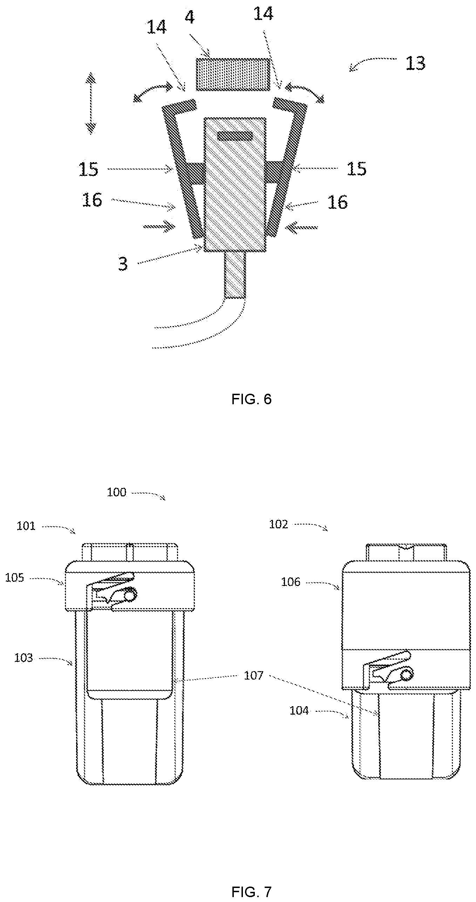

Dans un mode de réalisation schématisé en figure 6, les moyens de verrouillage (13) comprennent des moyens de retenus (14) agencés de manière à maintenir le capuchon (4) contre le connecteur (3) et des moyens de libération (15) activables grâce à un déclencheur (16). Un élément de sécurité peut être disposé de manière à retenir initialement le déclencheur (16). Sur cette figure conceptuelle, le pivot représente les moyens de libération (15), en appliquant une force contre le déclencheur (16) représenté par le bras des moyens de verrouillage (13), les moyens de retenus (14) en forme de crochet se déplacent pour libérer le capuchon (4). Dans ce mode de réalisation, l'ensemble connecteur capuchon peut également comprendre un ressort ou autre moyens d'entrainement comme une lame élastique qui permettrait d'éjecter ou déplacer le capuchon (4) lorsque les moyens de verrouillage (13) sont désactivés. Le capuchon (4) peut également comprendre de moyens de couplage. Ledit déclencheur (16) et/ou l'élément de sécurité peut être agencés sur et/ou dans le capuchon et/ou le connecteur.

Dans un mode de réalisation, les moyens de libération peuvent être activés en exerçant une force sur la face extérieure de l'extrémité distale du capuchon en direction du connecteur et les moyens d'entrainement peuvent être agencés de manière à éloigner ledit capuchon dudit connecteur au moins après activation des moyens de libération.

La description suivante s'applique à un mode de réalisation spécifique exposé par les figures 7 à 11:
La figure 7 présente le système de connecteur (100) qui comprend un premier ensemble (101) composé d'un connecteur de type femelle (103) également appelé premier connecteur et un capuchon (105) associé appelé également premier capuchon ainsi qu'un deuxième ensemble (102) composé d'un connecteur de type male (104) également appelé deuxième connecteur et un capuchon (106) associé appelé également deuxième capuchon.

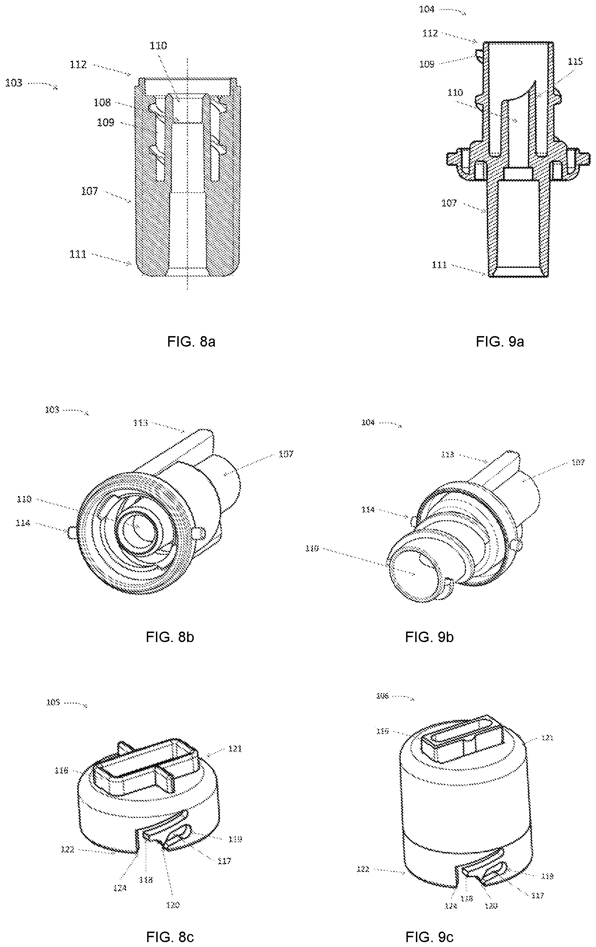

La figure 8a représente une coupe transversale du premier connecteur (103) composé d'un corps (107) traversé par un passage (110). L'extrémité proximale (111) du connecteur (103) est reliée de façon permanente à une ligne fluidique (non exposé). Le passage (110) peut être fermé grâce à l'aide d'une membrane (108) qui pourra être percée, déchirée et/ou ouverte par un autre connecteur, système ou dispositif.

La figure 9a représente une coupe transversale du deuxième connecteur (104) composé d'un corps (107) traversé par un passage (110). L'extrémité proximale (111) du connecteur (104) est reliée de façon permanente à une ligne fluidique (non exposé). Ledit passage (110) du deuxième connecteur (104) peut comprendre un élément cylindrique (115) qui peut entrer dans le passage (110) du premier connecteur (103). Ledit élément cylindrique (115) peut comprend une extrémité pointu de manière à percer ou ouvrir la membrane (108) du premier connecteur (103).

Les figures 8b et 9b représentent une vue en perspective des connecteurs (103, 104)). Lesdits connecteurs (103, 104) peuvent comprendre des moyens d'appréhension faisant saillie sur leur corps (107) afin de former des ailettes (113) qui permettent de faciliter la manipulation desdits connecteurs (103, 104). Lesdits connecteurs (103, 104) peuvent en outre comprendre des ergots (114) façonné de manière à coopérer avec les moyens de verrouillage.

Les figures 8c et 9c exposent une vue en perspectives de capuchons (105, 106) desdits connecteurs (103, 104). Le capuchon (105, 106) comprend des moyens de couplage (116). Dans un mode de réalisation, ledit capuchon (105, 106) comprend les moyens de verrouillage qui peuvent être composé d'au moins une fente (117) s'étendant de l'extrémité proximale (122) du capuchon jusqu'à une butée (119). Ladite fente (117) peut être rectiligne et/ou incurvée. L'épaisseur d'au moins une fente (117) est au moins égale à la largeur de l'ergot (114) de sorte que l'ergot (114) puisse coulisser à l'intérieur de ladite fente (117). Ladite butée (119) peut être façonnée de manière à bloquer au moins temporairement ledit ergot (114). Ladite fente peut comprendre ou être composé d'une lame élastique (118) pouvant disposer d'un élément (120) faisant saillie de manière à bloquer ledit ergot (114) du connecteur (103, 104) entre l'élément (120) faisant saillie et ladite butée (119). Ladite fente (117) peut contenir une deuxième butée (124) opposée à la première butée (119) et disposée proche de l'extrémité proximale (122).

Dans un autre mode de réalisation, les ergots peuvent être agencés sur le capuchon et les moyens de verrouillage sur le connecteur.

Dans un mode de réalisation, les moyens de verrouillage peuvent comprendre un système de filetage disposé sur les capuchons (105, 106) et/ou les connecteurs (103, 104); ledit système de filetage étant configuré de manière à se coupler et peut être composé d'au moins un ergot, d'un filet et/ou d'une multitude de filet.

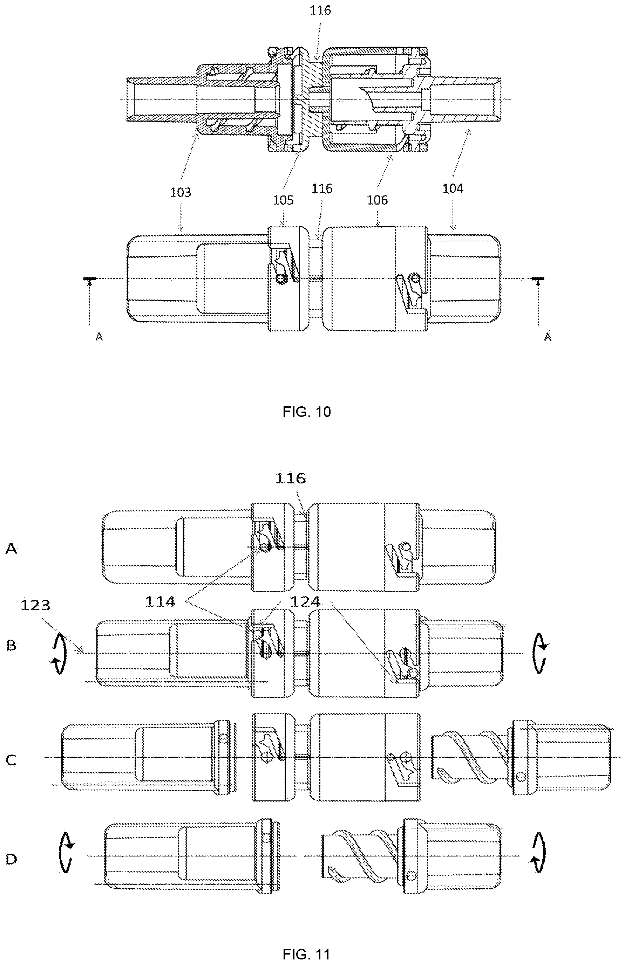

Dans la figure 10, les deux capuchons (105, 106) sont montés et verrouillés sur leur connecteur (103, 104) respectif. Les deux capuchons sont couplés l'un à l'autre grâce aux moyens de couplage (116).

La figure 11 montre la manipulation nécessaire pour décapuchonner et connecter les connecteurs. En phase A, les deux connecteurs (103, 104) munis de leur capuchon (105, 106) respectif sont rapprochés l'un de l'autre afin de coupler les deux capuchons (105, 106) ensemble. Les ergots (114) sont durant cette phase contre la butée (119) de la fente (117). En phase B, le praticien fait pivoter sur l'axe (123) longitudinal formé par le système. Les premier et deuxième connecteurs se font faces, ainsi les mains maintenant les corps des connecteurs doivent effectuer une rotation permettant de déverrouiller les capuchons. Les capuchons (105, 106) étant maintenus grâce au moyen de couplage, sont solidaires et subissent les contraintes de rotation des connecteurs. Ainsi, les ergots (114) coulissent à travers la fente (117) jusqu'à l'élément (120) faisant saillie sur la lame élastique (118). Selon ce mode de réalisation, une force supplémentaire peut être appliquée afin de faire fléchir la lame élastique (118) pour qu'au moins un ergot (114) puisse être libéré de la fente (118). La deuxième butée (124) permet de retenir l'ergot (114) libéré afin que le processus de libération puisse s'effectuer sur l'ergot (114) de l'autre capuchon (105, 106). En phase C, les capuchons (105, 106) sont déverrouillés de leur connecteur (103, 104). Il suffit alors d'éloigner les deux connecteurs (103, 104) et de laisser tomber les capuchons (105, 106). Dans un mode de réalisation, la lame élastique (118) agit également en tant que moyens d'entrainement afin de repousser le capuchon (105, 106) de son connecteur (103, 104) voir de l'éjecter. En phase D, les deux connecteurs (103, 104) sont rapprochés l'un à l'autre et connectés. Dans un mode de réalisation, les connecteurs (103, 104) comprennent un système de filetage façonné de manière à se coupler. La connexion peut être effectuée par une rotation de sens inverse de celle de l'étape B. Dans un mode de réalisation préférée, l'angle de rotation de déverrouillage des capuchons peut être est substantiellement égal à l'angle de rotation pour la connexion des connecteurs mais de sens inverse. Dans un mode de réalisation, ledit système de filetage qui permet de coupler les connecteurs est également utilisé pour coupler les connecteurs à leurs capuchons respectifs.

Autrement dit, l'ensemble connecteur/capuchon est conçu pour éviter que l'utilisateur ne touche les capuchons pour les ôter. De plus, afin de limiter la contamination indirecte, l'ensemble connecteur/capuchon est conçu pour forcer à décapuchonner avant de connecter les connecteurs entre eux de manière à éloigner les capuchons du passage d'écoulement. Il s'agit ainsi d'une double mesure de précaution qui diminue de façon substantielle les risques de contamination directe et indirecte.

Dans un mode de réalisation, l'élément (120) faisant saillie sur la lame élastique (118) est façonné de manière à ce que l'ergot libéré (c'est à dire capuchon déverrouillé de son connecteur) ne puisse revenir dans sa position initiale contre la première buté (119). Dit autrement, une fois déverrouillé, l'élément (120) faisant saillie sur la lame élastique (118) empêche que le capuchon (105, 106) ne puisse être reverrouillé sur son connecteur (103, 104). Par exemple, la forme dudit élément (120) peut empêcher le chemin inverse ou la lame élastique (118) peut se rompre de sorte que le capuchon (105, 106) ne puisse plus tenir ou être reverrouillé sur le connecteur (103, 104).

Ce mode de réalisation est particulièrement pertinent pour éviter de toucher le capuchon. En effet, une manipulation simple et intuitive permet le décapuchonnage des connecteurs. De plus, sans avoir à changer de les doigts de position sur le connecteur, il est possible de connecter de façon sure et propre les connecteurs.

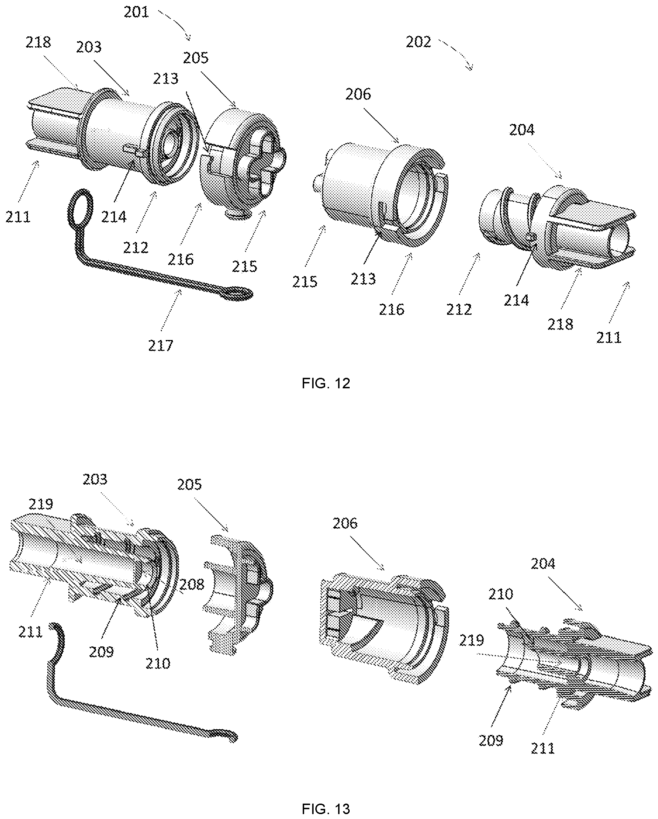

Dans un mode de réalisation décrit par les figures 12 à 14, chaque connecteur comprend :
- des moyens de connexion fluidique (210),
- des moyens de connexion physique (209),
- des capuchons (205, 206).

Dans un mode de réalisation préférée, pour chaque ensemble connecteur / capuchon (201, 202), les moyens de connexion fluidique comprennent une lumière définissant un passage d'écoulement de fluide (219). Ce passage d'écoulement est en connexion fluidique avec un tube (220) qui s'étend au-delà de l'extrémité proximale du connecteur (211). Les moyens de connexion physique (209) entourent au moins partiellement les moyens de connexion fluidique (210) de sorte les moyens de connexion fluidique (210) soient au moins partiellement protégés par les moyens de connexion physique (209). Les moyens de connexion fluidique ont pour objectif de permettre créer un passage d'écoulement de fluide continu entre le premier et deuxième connecteur. Et les moyens de connexion physique assurent la bonne connexion et le maintien de la connexion entre les deux connecteurs. Préférentiellement, les moyens de connexion fluidique (210) et les moyens de connexion physique (209) sont solidement et définitivement fixés au corps (207) du connecteur. Le corps (207) du connecteur peut être formé d'une seule pièce comprenant les moyens de connexion fluidique (210) et physique (209). Le capuchon (205, 206) est façonné de manière à protéger et/ou entourer les moyens de connexion fluidique (210) ainsi que les moyens de connexion physique (209).

Ainsi, le mode de réalisation des figures 12 à 14 permet de nombreux avantages tant au niveau garanti de la stérilité qu'au niveau coût de fabrication et utilisation du connecteur.

Dans un mode de réalisation, le système comprend un connecteur définissant des moyens de connexion physique de type male alors que le connecteur compatible comprend des moyens de connexion physique de type femelle. De même, le système comprend un connecteur définissant des moyens de connexion fluidique de type male alors que le connecteur compatible comprend des moyens de connexion fluidique de type femelle. Préférentiellement, les connecteurs comprennent des moyens de connexion physique et fluidique de même type.

Au moins un des moyens de connexion physique fait saillie par rapport au moyen de connexion fluidique et comprend une surface interne définissant une enceinte de sécurité. Autrement dit, au moins un des deux moyens de connexion physique entourent et dépassent les moyens de connexion fluidique afin de définir une enceinte de sécurité. Préférentiellement, la surface externe des moyens de connexion physique de type femelle est en contact directe avec l'environnement extérieur.

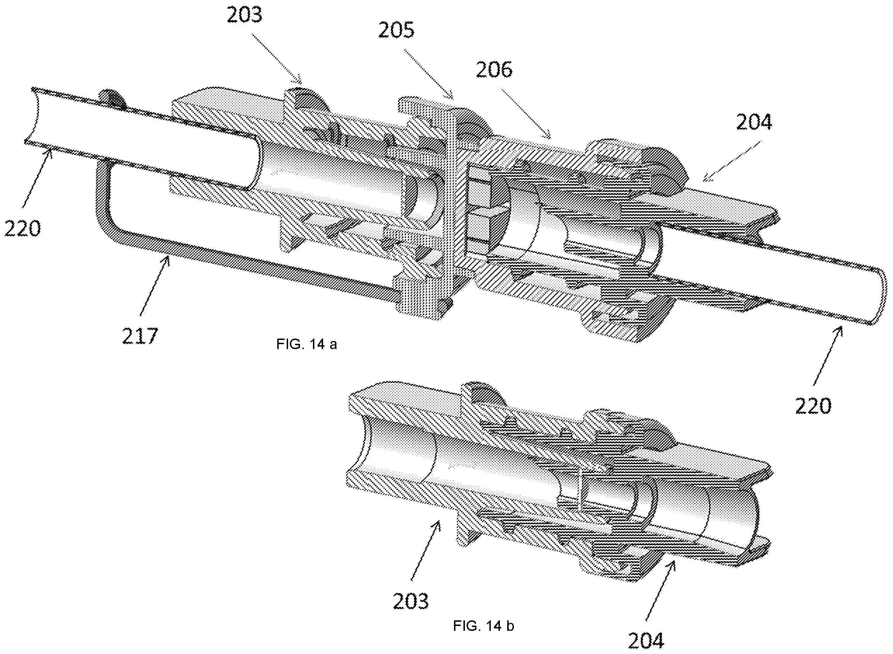

Ainsi, comme le montre la figure 14 b, lorsque les connecteurs sont connectés, les différents moyens de connexion se télescopent entre eux afin d'améliorer l'étanchéité du système de connexion et/ou afin de garantir la stérilité du passage d'écoulement. Ainsi, les moyens de connexion physique du premier connecteur (203) entourent et protègent les moyens de connexion physique du deuxième connecteur (204) qui, à son tour, entourent et protègent les moyens de connexion fluidique du premier connecteur (203) qui, à son tour, entourent et protègent les moyens de connexion fluidique du deuxième connecteur (204). Autrement dit les moyens de connexion fluidique de type mal entrent dans les moyens de connexion fluidique de type femelle qui entrent dans les moyens de connexion fluidique de type male qui entrent dans les moyens de connexion de type femelle.

Dans un mode de réalisation, le capuchon (205) du premier connecteur (203) peut comprendre un élément interne faisant saillie permettant d'entourer au moins partiellement les moyens de connexion fluidique (210) du premier connecteur (203).

Dans un mode de réalisation, les moyens de connexion physique (209) comprennent un système de filetage compatible disposé sur et/ou dans le corps des connecteurs. En particulier, le système de filetage du premier connecteur est agencé sur une partie intérieure de ses moyens de connexion physique et le système de filetage du deuxième connecteur est agencé sur une partie extérieure de ses moyens de connexion physique.

Afin d'éviter que les capuchons ne tombent au sol lorsque ces derniers sont ôté des connecteurs, au moins un des couples connecteur / capuchon comprend un dispositif de retenu (217) reliant un connecteur à son capuchon. Si les moyens de couplage des capuchons le permettent, l'autre capuchon ne disposant pas de dispositif de retenu est maintenu au capuchon relié auxdit dispositif de retenu (217). Toutefois, il est important que ce dispositif de retenu (217) soit conçu de manière à permettre au capuchon de s'éloigner suffisamment des connexions fluidiques, autrement dit de l'enceinte de sécurité.

Selon un mode de réalisation, les deux connecteurs compatibles (203, 204) sont fabriqués d'une matière ne comportant pas les mêmes caractéristiques physiques. Par exemple, le deuxième connecteur est fabriqué à partir d'une matière relativement plus dure que le premier connecteur. Cela permet de créer des contraintes augmentant les forces de frottement afin de garantir un bon maintient et/ou une certaine étanchéité lorsque les connecteurs sont connecté entre eux.

Selon un mode de réalisation, le connecteur et le capuchon sont fabriquées d'une matière ne comportant pas les mêmes caractéristiques physiques. Par exemple, le deuxième connecteur est relativement plus dur que son capuchon. Cela permet de créer des contraintes augmentant les forces de frottement afin de garantir un bon maintient lorsque le capuchon est verrouillé sur son connecteur.

Ainsi, selon ce même principe, le premier connecteur est fabriqué d'une matière relativement plus molle que son capuchon et que le deuxième connecteur. Le deuxième connecteur est fabriqué d'une matière relativement plus dure que son capuchon et que le premier connecteur. Pour faciliter et améliorer la coopération des moyens de couplage des capuchons, le capuchon du premier connecteur peut être fabriqué d'une matière relativement plus dure que le capuchon du deuxième connecteur. De même, le premier connecteur et le capuchon du deuxième connecteur (réciproquement : le deuxième connecteur et le capuchon du premier connecteur) peuvent avoir des caractéristiques physiques identique et ils peuvent être caractérisé selon un niveau de dureté substantiellement égal.

## Revendications

1. Système de connecteur (12) comprenant deux ensembles connecteur/capuchon (1) dans lequel les deux connecteurs (3) sont conçus pour relier deux lignes fluidiques (2), chaque connecteur comprenant :
• un corps (9) conçu pour être appréhendé par un utilisateur ;
• une extrémité distale (5) destinée à être connecté à un autre connecteur (3) ;
• une extrémité proximale (6) reliée à une ligne fluidique (2) ;
• un passage d'écoulement de fluide (219) en connexion fluidique avec ladite ligne fluidique (2) et protégé par un capuchon (4) disposé au niveau de l'extrémité distale (5) du connecteur (3) et empêchant la connexion physique des connecteurs (3) tant que les capuchons (5) protègent le passage d'écoulement de fluides (219);
• des moyens de connexion physique (209) permettant de maintenir deux connecteurs (3) connectés entre eux ;
**caractérisé en ce que**
l'ensemble connecteur/capuchon (1) comprend des moyens de verrouillage (13) conçus pour maintenir au moins temporairement et solidement les capuchons (4) aux connecteurs (3) et comprenant un mécanisme pour déverrouiller les moyens de verrouillage (13) afin de décapuchonner le connecteur (3) ;
les capuchons (4) comprennent des moyens de couplage (11) agencés de manière à coupler les capuchons (4) desdits connecteurs (3) avec des éléments comportant également des moyens de couplage (11) au moins durant la désactivation des moyens de verrouillage (13); et
le mécanisme de déverrouillage est configuré pour être actionné par l'utilisateur quand les capuchons (4) sont couplés à des éléments comportant également des moyens de couplage (11) et fixés à leur connecteur (3) respectif par les moyens de verrouillage (13), afin de désactiver les moyens de verrouillage (13) permettant de décapuchonner les connecteurs (3) sans que l'utilisateur n'ait à toucher les capuchons (4).

2. Système selon la revendication 1, dans lequel chaque capuchon (4) comprend :
• une extrémité distale (7) destiné à être couplé à un autre capuchon (4),
• une extrémité proximale (8) destiné à être couplé au connecteur (3).

3. Système selon l'une des précédentes revendications, dans lequel les capuchons (4) s'étendent de manière à protéger les moyens de connexion physique (209) des connecteurs (3).

4. Système selon l'une des précédentes revendications, dans lequel les moyens de connexion fluidique (210) et physique (209) sont agencés de manière à être connecté en même temps.

5. Système selon l'une des précédentes revendications, dans lequel les moyens de couplage (11) permettent de décapuchonner les connecteurs (3).

6. Système selon l'une des précédentes revendications, dans lequel les connecteurs (3) ne peuvent être décapuchonnés que lorsque les capuchons (4) sont couplés grâce aux moyens de couplage (11).

7. Système selon l'une des précédentes revendications, dans lequel au moins un des moyens de couplage (11) est agencé de manière à maintenir au moins temporairement lesdits capuchons (4) couplés de façon solidaire.

8. Système selon l'une des précédentes revendications, dans lequel au moins un des connecteurs (3) et/ou au moins un des capuchons (4) comprend des moyens d'entrainement de manière à éloigner au moins partiellement le capuchon (4) du connecteur (3) lorsque les moyens de verrouillage (13) sont désactivés.

9. Système selon l'une des précédentes revendications, dans lequel les moyens de couplage (11) comportent un système de codage de manière à ce que seuls les codages compatibles permettent aux capuchons (4) de se coupler l'un à l'autre.

10. Système selon la revendication précédente, dans lequel les moyens de couplage (11) d'un capuchon (4) sont compatibles avec les moyens de couplage (11) d'un seul capuchon (4) ou de plusieurs capuchons (4) de sorte que certains couples connecteurs/capuchons soient compatibles avec une série d'autres couples connecteurs/capuchons.

11. Système selon l'une des précédentes revendications, dans lequel lesdits moyens de verrouillage (13) comprennent au moins un ergot (114).

12. Système selon l'une des précédentes revendications, dans lequel lesdits moyens de verrouillage (13) comprennent au moins une fente (117).

13. Système selon l'une des précédentes revendications, dans lequel les moyens de verrouillage (13) comprennent au moins un élément de sécurité empêchant toute désactivation non intentionnelle des moyens de verrouillage (13).

14. Système selon la revendication précédente, dans lequel l'élément de sécurité comprend une lame élastique (118) sur lequel un élément faisant saillit (120) est façonné de manière à bloquer au moins temporairement lesdits moyens de verrouillage (13).

15. Système selon la revendication 13, dans lequel l'élément de sécurité est façonné de manière à empêcher de reverrouiller le connecteur (3) après avoir été déverrouillé.

## Patentansprüche

1. Steckverbindersystem (12), welches zwei Steckverbinder-Schutzkappen-Einheiten (1) umfasst, wobei die zwei Steckverbinder (3) dafür eingerichtet sind, zwei Fluidleitungen (2) zu verbinden, wobei jeder Steckverbinder umfasst:
• einen Körper (9), der dafür eingerichtet ist, von einem Benutzer ergriffen zu werden;
• ein distales Ende (5), das dazu bestimmt ist, mit einem anderen Steckverbinder (3) verbunden zu werden;
• ein proximales Ende (6), das mit einer Fluidleitung (2) verbunden ist;
• einen Fluidströmungsdurchgang (219), der mit der Fluidleitung (2) in Fließverbindung steht und durch eine Schutzkappe (4) geschützt ist, die am distalen Ende (5) des Steckverbinders (3) angeordnet ist und die physische Verbindung der Steckverbinder (3) verhindert, solange die Schutzkappen (5) den Fluidströmungsdurchgang (219) schützen;
• Mittel zur physischen Verbindung (209), die es ermöglichen, zwei Steckverbinder (3) miteinander verbunden zu halten;
**dadurch gekennzeichnet, dass**
die Steckverbinder-Schutzkappen-Einheit (1) Verriegelungsmittel (13) umfasst, die dafür eingerichtet sind, die Schutzkappen (4) wenigstens vorübergehend und fest an den Steckverbindern (3) zu halten, und einen Mechanismus zum Entriegeln der Verriegelungsmittel (13) umfassen, um die Schutzkappe vom Steckverbinder (3) zu entfernen;
die Schutzkappen (4) Kopplungsmittel (11) umfassen, die dafür ausgelegt sind, wenigstens während der Deaktivierung der Verriegelungsmittel (13) die Schutzkappen (4) der Steckverbinder (3) mit Elementen zu koppeln, die ebenfalls Kopplungsmittel (11) aufweisen; und
der Entriegelungsmechanismus dafür eingerichtet ist, vom Benutzer betätigt zu werden, wenn die Schutzkappen (4) mit Elementen gekoppelt sind, die ebenfalls Kopplungsmittel (11) aufweisen, und an ihrem jeweiligen Verbinder (3) durch die Verriegelungsmittel (13) befestigt sind, um die Verriegelungsmittel (13) zu deaktivieren, was es ermöglicht, die Schutzkappen von den Steckverbindern (3) zu entfernen, ohne dass der Benutzer die Schutzkappen (4) berühren muss.

2. System nach Anspruch 1, wobei jede Schutzkappe (4) umfasst:
• ein distales Ende (7), das dazu bestimmt ist, mit einer anderen Schutzkappe (4) gekoppelt zu werden,
• ein proximales Ende (8), das dazu bestimmt ist, mit dem Verbinder (3) gekoppelt zu werden.

3. System nach einem der vorhergehenden Ansprüche, wobei sich die Schutzkappen (4) derart erstrecken, dass sie die Mittel zur physischen Verbindung (209) der Steckverbinder (3) schützen.

4. System nach einem der vorhergehenden Ansprüche, wobei die Mittel zur Fließverbindung (210) und zur physischen Verbindung (209) dafür ausgelegt sind, gleichzeitig verbunden zu werden.

5. System nach einem der vorhergehenden Ansprüche, wobei die Kopplungsmittel (11) ermöglichen, die Schutzkappen von den Steckverbindern (3) zu entfernen.

6. System nach einem der vorhergehenden Ansprüche, wobei die Schutzkappen nur dann von den Steckverbindern (3) entfernt werden können, wenn die Schutzkappen (4) mithilfe der Kopplungsmittel (11) gekoppelt sind.

7. System nach einem der vorhergehenden Ansprüche, wobei wenigstens eines der Kopplungsmittel (11) so gestaltet ist, dass es die Schutzkappen (4) wenigstens vorübergehend fest gekoppelt hält.

8. System nach einem der vorhergehenden Ansprüche, wobei wenigstens einer der Steckverbinder (3) und/oder wenigstens eine der Schutzkappen (4) Antriebsmittel umfasst, um die Schutzkappe (4) wenigstens teilweise vom Steckverbinder (3) zu entfernen, wenn die Verriegelungsmittel (13) deaktiviert werden.

9. System nach einem der vorhergehenden Ansprüche, wobei die Kopplungsmittel (11) ein Codiersystem aufweisen, so dass nur die kompatiblen Codierungen ermöglichen, dass die Schutzkappen (4) miteinander gekoppelt werden.

10. System nach dem vorhergehenden Anspruch, wobei die Kopplungsmittel (11) einer Schutzkappe (4) mit den Kopplungsmitteln (11) nur einer Schutzkappe (4) oder von mehreren Schutzkappen (4) kompatibel sind, so dass gewisse Paare Steckverbinder/Schutzkappen mit einer Reihe von anderen Paaren Steckverbinder/Schutzkappen kompatibel sind.

11. System nach einem der vorhergehenden Ansprüche, wobei die Verriegelungsmittel (13) wenigstens einen Vorsprung (114) umfassen.

12. System nach einem der vorhergehenden Ansprüche, wobei die Verriegelungsmittel (13) wenigstens einen Schlitz (117) umfassen.

13. System nach einem der vorhergehenden Ansprüche, wobei die Verriegelungsmittel (13) wenigstens ein Sicherheitselement umfassen, das jede versehentliche Deaktivierung der Verriegelungsmittel (13) verhindert.

14. System nach dem vorhergehenden Anspruch, wobei das Sicherheitselement eine elastische Lamelle (118) umfasst, an welche ein vorstehendes Element (120) so angeformt ist, dass die Verriegelungsmittel (13) wenigstens vorübergehend blockiert werden.

15. System nach Anspruch 13, wobei das Sicherheitselement so geformt ist, dass es ein erneutes Verriegeln des Steckverbinders (3), nachdem er entriegelt worden ist, verhindert.

## Claims

1. Connector system (12) comprising two connector/cap assemblies (1) wherein the two connectors (3) are designed to connect two fluid lines (2), each connector comprising:
• a body (9) designed to be held by a user;
• a distal end (5) intended to be connected to another connector (3);
• a proximal end (6) connected to a fluid line (2);
• a fluid flow passage (219) in fluid connection with said fluid line (2) and protected by a cap (4) disposed at the distal end (5) of the connector (3) and preventing physical connection of the connectors (3) while the caps (5) are protecting the fluid flow passage (219);
• physical connection means (209) making it possible to keep two connectors (3) connected to each other;
**characterized in that**
the connector/cap assembly (1) comprises locking means (13) designed to at least temporarily and firmly hold the caps (4) to the connectors (3) and comprising a mechanism for unlocking the locking means (13) in order to uncap the connector (3);
the caps (4) comprise coupling means (11) adapted to couple the caps (4) of said connectors (3) to elements also having coupling means (11) at least during deactivation of the locking means (13) and;
the unlocking mechanism is configured to be actuated by the user when the caps (4) are coupled to elements also comprising coupling means (11) and fixed to their respective connector (3) by the locking means (13), in order to deactivate the locking means (13) such that the connectors (3) can be uncapped without the user having to touch the caps (4).

2. System according to Claim 1, wherein each cap (4) comprises:
• a distal end (7) intended to be coupled to another cap (4);
• a proximal end (8) intended to be coupled to the connector (3).

3. System according to either one of the preceding claims, wherein the caps (4) extend in such a manner as to protect the physical connection means (209) of the connectors (3).

4. System according to one of the preceding claims, wherein the fluid connection means (210) and the physical connection means (209) are arranged so as to be connected at the same time.

5. System according to one of the preceding claims, wherein the coupling means (11) enable uncapping of the connectors (3).

6. System according to one of the preceding claims, wherein the connectors (3) can be uncapped only when the caps (4) are coupled by the coupling means (11).

7. System according to one of the preceding claims, wherein at least one of the coupling means (11) is arranged so as to keep said caps (4) firmly coupled at least temporarily.

8. System according to one of the preceding claims, wherein at least one of the connectors (3) and/or at least one of the caps (4) comprises drive means for at least partly moving the cap (4) away from the connector (3) when the locking means (13) are deactivated.

9. System according to one of the preceding claims, wherein the coupling means (11) comprise a coding system so that only compatible codings enable coupling of the caps (4) to each other.

10. System according to the preceding claim, wherein the coupling means (11) of a cap (4) are compatible with the coupling means (11) of only one cap (4) or of a plurality of caps (4) so that certain connector/cap pairs are compatible with a series of other connector/cap pairs.

11. System according to one of the preceding claims, wherein said locking means (13) comprise at least one lug (114).

12. System according to one of the preceding claims, wherein said locking means (13) comprise at least one slot (117).

13. System according to one of the preceding claims, wherein the locking means (13) comprise at least one safety element preventing unintentional deactivation of the locking means (13).

14. System according to the preceding claim, wherein the safety element comprises a leaf spring (118) on which a projecting element (120) is fashioned so as to immobilize said locking means (13) at least temporarily.

15. System according to Claim 13, wherein the safety element is fashioned so as to prevent the connector (3) being locked again once unlocked.
